# EUROPEAN PATENT SPECIFICATION

(11) **EP 0 564 059 B1**
(45) Date of publication and mention of the grant of the patent: **02.11.1995**
(21) Application number: 93250079.6
(22) Date of filing: 12.03.1993
(51) Int. Cl.: A61B 17/06

(54) **Surgical needle**
Chirurgische Nadel
Aiguille chirurgicale

(30) Priority: 14.03.1992 DE 4208242
(43) Date of publication of application: 06.10.1993
(73) Proprietor: ETHICON INC., Somerville, New Jersey 08876-0151 (US)
(72) Inventor: Brunken, Dieter, W-2359 Hüttblek (DE)
(74) Representative: UEXKÜLL & STOLBERG

(56) References cited:
- EP-A- 0 263 347
- DE-C- 3 841 443
- US-A- 3 840 015
- US-A- 4 905 695
- US-A- 4 959 068

## Description

The invention relates to a surgical needle with a suture material or thread fixed to the end opposite to the puncturing tip.

Such surgical needles or suture needles are generally known and are normally made from a corrosion-resistant metal, preferably chrome-nickel steel. Bare or surface-untreated needles are used for minor surgical operations. However, in the case of major surgery, of late so-called coloured or dulled needles have been used, such as are e.g. described in US Patent 4,959,068. This colouring or dulling takes place either chemically by pickling or etching or electrolytically by a corresponding anodic or cathodic treatment, optionally with polarity reversal or by alternating current. A special form of pickling is so-called dull pickling. In certain cases stoving lacquers are also used for colouring or dulling. As a result of the so-called colouring or dulling of the surgical needle, the latter does not reflect under powerful OP-light and irritate the surgeon, which is particularly advantageous in major operations.

Despite the popularity of such coloured or dulled needles, it has proved disadvantageous that particularly with yellow to dark red and brown coloured or dulled needles the surgeon is unable to precisely detect in the similarly coloured tissue or operating field the point of perforation of the needle, i.e. the needle tip and the area following onto the same, which makes it more difficult to precisely insert a suture not only in microsurgery, but in general surgical operations.

The problem of the invention is to eliminate this disadvantage and make available a surgical needle, which has both the advantage of coloured or dulled needles, i.e. freedom from glare, whilst simultaneously permitting a precise point-sized insertion of the needle into the corresponding tissue.

Thus, for solving the set problem, a surgical needle of the aforementioned construction is proposed, which is constructed in such a way that the puncturing tip and the area of the needle following onto the latter is made up to approximately 50% of its length from bare or surface-untreated metal, whereas the surface of the remaining part of the needle and up to the thread shoulder is chemically or electrolytically or by means of a coating dulled or coloured in a continuous manner or with minor interruptions.

It has surprisingly been found that with a needle according to the invention in this form and contrary to the previously held opinion that the entire needle must be dulled or coloured, it is still possible to operate in a glare-free manner and that with the relatively short tip of the bare or surface-untreated metal it is possible to insert a suture much more accurately.

Preferably the bare or surface-untreated area represents less than 25% of the needle length, said value also being dependent on the needle size or length and in absolute terms can in particular be 4 to 10 mm.

In place of a continuous dulling or colouring of the remaining part of the needle, the latter can be dulled or coloured with minor interruptions and in particular this coloured or dulled area can be dulled or coloured in ring form or in speckled form. The bare gaps in the only zonally dulled or coloured area are preferably no larger than 1 to 2 mm.

The invention is described in greater detail hereinafter relative to the drawings, wherein show:

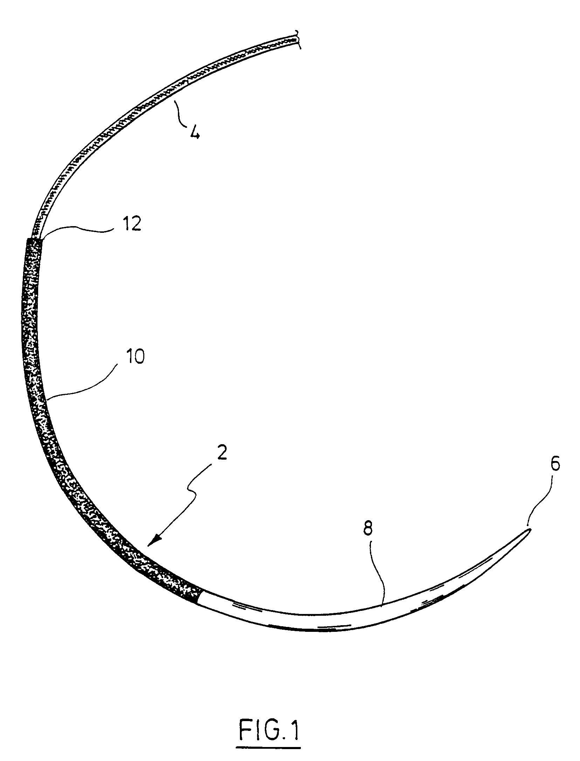

- Fig. 1: a larger-scale representation of the surgical needle according to the invention with a continuously dulled or coloured area.
- Fig. 2: a representation identical to fig. 1 with an only zonally dulled or coloured area in ring form.
- Fig. 3: a representation identical to fig. 2 with a dulled or coloured area in speckled form.

The needle 2 is a standard semicircular round body needle. However, the needle can have any other random configuration and can e.g. be a blunt round body needle, a cutting needle or a spatula needle. At one end of the needle is located the perforation or puncturing tip 6, which can also be constructed as a microtip, whereas at the opposite end of the needle it is possible to see the thread shoulder 12 with the thread 4. The tip 6 and the area 8 following onto it and which represents approximately less than 50% of the needle length, is bare or surface-untreated, whereas the remaining part 10 is continuously dulled or coloured either chemically or electrolytically on the one hand, or by means of a coating on the other.

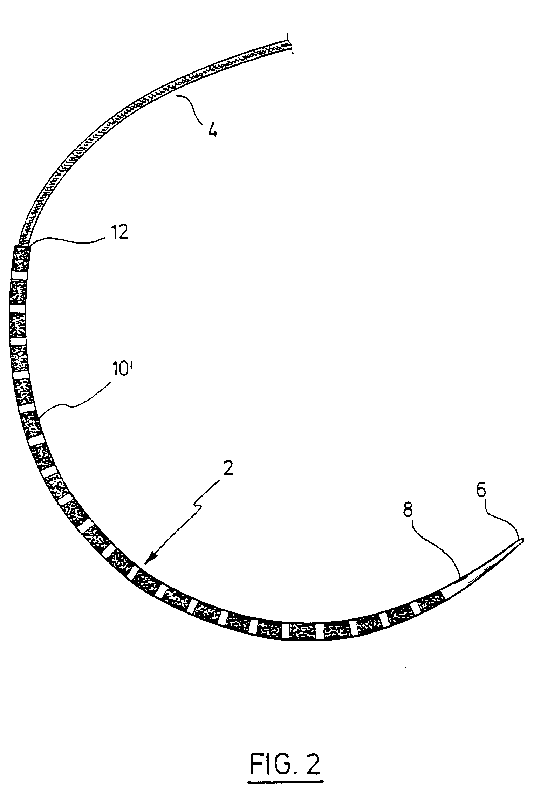

In the case of fig. 2 the dulled or coloured area 10' is dulled or coloured in ring form. The randomly wide, dulled or coloured rings are spaced by approximately 1 to 2 mm. This spacing is sufficient to avoid any glare, but is also sufficient to make it possible to adequately detect the further contour of the needle in the operating field.

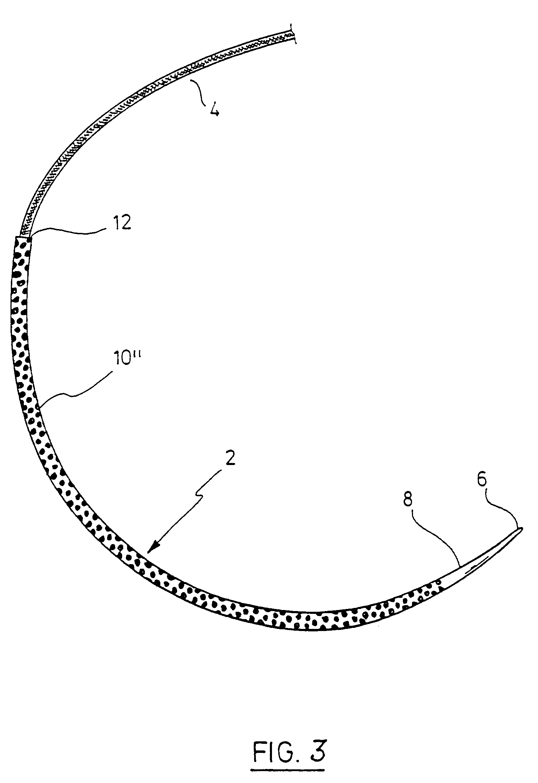

In the needle shown in fig. 3 the dulled or coloured area 10'' is speckled and the spacings of the coloured or dulled speckles must be no greater than 1 to 2 mm.

In all the needles shown the colour of the dulled or coloured areas preferably coincides with the colour of the thread 4, which leads to the further advantage that the coding colour of the thread can be detected by intuition in the case of needles in a pack.

## Claims

1. Surgical needle with a suture material or thread fixed to the end opposite to the perforation tip, characterized in that the puncturing tip (6) and the area (8) of the needle (2) following onto said tip is made up to approximately 50% of its length from bare or surface-untreated metal, whereas the surface of the remaining part (10) of the needle and up to the thread shoulder (12) is chemically or electrolytically or by means of a coating dulled or coloured in a continuous manner or with minor interruptions.

2. Needle according to claim 1, characterized in that the bare or surface-untreated area (6,8) of the needle is less than 25% of the needle length.

3. Needle according to claim 1, characterized in that the bare or surface-untreated area, measured from the needle tip is 4 to 10 mm.

4. Needle according to claim 1, characterized in that the part (10) of the needle dulled or coloured with minor interruptions (14,16) is dulled or coloured in ring form (10') or in speckled form (10'') and both in the tip area (6,8) and also between the dulled or coloured areas is bare or surface-untreated.

## Patentansprüche

1. Chirurgische Nadel mit einem an dem der Einstichspitze entgegengesetzten Ende befestigten Nahtmaterial oder Faden, dadurch gekennzeichnet, daß die Einstichspitze (6) und der sich an diese anschließende Bereich (8) der Nadel (2) bis zu etwa 50% ihrer Länge aus blankem oder oberflächlich unbehandeltem Metall besteht, während die Oberfläche des restlichen Teiles (10) der Nadel bis zum Fadenansatz (12) chemisch oder elektrolytisch oder durch einen Überzug durchgehend oder mit geringfügigen Unterbrechungen mattiert oder gefärbt ist.

2. Nadel nach Anspruch 1, dadurch gekennzeichnet, daß der blanke oder oberflächlich unbehandelte Bereich (6, 8) der Nadel weniger als 25% der Nadellänge beträgt.

3. Nadel nach Anspruch 1, dadurch gekennzeichnet, daß der blanke oder oberflächlich unbehandelte Bereich von der Nadelspitze gemessen 4 bis 10 mm beträgt.

4. Nadel nach Anspruch 1, dadurch gekennzeichnet, daß der mit geringfügigen Unterbrechungen (14, 16) mattierte oder gefärbte Teil (10) der Nadel in Ringform (10') oder in Sprenkelform (10'') mattiert oder gefärbt ist und sowohl im Spitzenbereich (6, 8) als auch zwischen den mattierten oder gefärbten Bereichen blank bzw. oberflächlich unbehandelt ist.

## Revendications

1. Aiguille chirurgicale avec un matériau de suture, ou fil, fixé à l'extrémité opposée à la pointe de perforation, caractérisée en ce que la pointe de percement (6) et la zone (8) de l'aiguille (2), à la suite sur ladite pointe, est réalisée sur jusqu'à approximativement 50% de sa longueur, à partir de métal à nu ou de surface non traitée, tandis que la surface de la partie restante (10) de l'aiguille, et jusqu'à l'épaulement du fil (12), est chimiquement ou de façon électrolytique ou au moyen d'un revêtement, ternie ou colorée de manière continue ou avec de petites interruptions.

2. Aiguille selon la revendication 1, caractérisée en ce que la zone à nu ou de surface non traitée (6, 8) de l'aiguille est inférieure à 25% de la longueur d'aiguille.

3. Aiguille selon la revendication 1, caractérisée en ce que la zone à nu ou de surface non traitée, mesurée à partir de la pointe d'aiguille, est de 4 à 10 mm.

4. Aiguille selon la revendication 1, caractérisée en ce que la partie (10) de l'aiguille ternie ou colorée avec de petites interruptions (14, 16), est ternie ou colorée sous forme d'anneaux (10') ou sous forme tachetée (10'') et, à la fois dans la zone de la pointe (6, 8) et également entre les zones ternies ou colorées, est à nu ou de surface non traitée.
